(19) Europäisches Patentamt
European Patent Office
Office européen des brevets

(11) EP 0 826 769 A1

(12) **DEMANDE DE BREVET EUROPEEN**

(43) Date de publication:
**04.03.1998 Bulletin 1998/10**

(51) Int Cl.[6]: **C11D 3/37**

(21) Numéro de dépôt: **97420144.4**

(22) Date de dépôt: **12.08.1997**

(84) Etats contractants désignés:
**AT BE CH DE DK ES FI FR GB GR IE IT LI LU MC NL PT SE**
Etats d'extension désignés:
**AL LT LV RO SI**

(30) Priorité: **26.08.1996 FR 9610585**

(71) Demandeur: **COATEX S.A.**
**F-69730 Genay (FR)**

(72) Inventeurs:
- **Kensicher, Yves**
**69380 Lozanne (FR)**
- **Suau, Jean-Marc**
**69480 Lucenay (FR)**

(54) **Agent compatible avec les tensio-actifs utilsés en détergence ou cosmétique**

(57) Utilisation de copolymères de formule générale :

$$(X)_a-(Y)_b-(Z)_c$$

dans laquelle

- X représente le motif monomérique à charge anionique
- Y représente le motif monomérique à charge non ionique
- Z représente le motif monomérique à charge cationique

avec a, pourcentage en poids par rapport à la masse totale des monomères est compris entre 95 et 15
avec b, pourcentage en poids par rapport à la masse totale des monomères compris entre 0 et 65
avec c, pourcentage en poids par rapport à la masse totale des monomères compris entre 5 et 60

dans le but d'obtenir des compositions détergentes ou cosmétiques stables.

EP 0 826 769 A1

## Description

L'invention concerne la compatibilité avec les tensioactifs présents dans les compositions détergentes ou cosmétiques, de copolymères comprenant au moins deux motifs monomériques dont le premier est composé d'un ou plusieurs monomères éthyléniques à charge anionique, le deuxième est composé d'un ou plusieurs monomères éthyléniques à charge cationique dont l'un au moins de ces monomères à charge cationique possède une structure tensioactive, et comprenant éventuellement un troisième motif qui est composé d'un ou plusieurs monomères éthyléniques à caractère non ionique.

De nos jours les formulations détergentes ou cosmétiques comprennent, de plus en plus souvent des modificateurs de rhéologie et/ou des agents antitartre en plus des tensioactifs anioniques, cationiques, amphotères ou non ioniques et éventuellement des builders du type borax, citrate, formiate de sodium ou sels d'acide faible, outre divers autres additifs tels que par exemple du propylène glycol, des azurants optiques et autres.

L'homme du métier doit alors faire face au problème de la compatibilité du modificateur de rhéologie qu'il soit dispersant ou épaississant et/ou de l'agent anti-tartre avec le tensioactif présent dans la formulation.

Jusqu'à aujourd'hui l'homme du métier ne disposait pas de solutions pour résoudre ce problème de compatibilité et devait choisir l'agent dispersant ou épaississant ou anti-tartre en fonction du tensio-actif présent dans la formulation détergente ou cosmétique.

Il a maintenant été découvert que l'utilisation de polymère amphotère, comprenant au moins deux motifs monomériques dont le premier est constitué d'un ou plusieurs monomères éthyléniques à charge anionique, le deuxième est constitué d'un ou plusieurs monomères éthyléniques à charge cationique, dont l'un au moins de ces monomères à charge cationique possède une structure tensioactive c'est-à-dire constituée d'une ou plusieurs chaînes latérales oxyalkylées et composées de groupements alkyle, aryle, alkylaryrle ou arylalkyle possédant au moins 8 atomes de carbone et comprenant éventuellement un troisième motif constitué d'un ou plusieurs motifs à caractère non ionique, permet de manière surprenante d'obtenir la compatibilité des différents constituants de la formulation détergente ou cosmétique c'est-à-dire d'obtenir des compositions détergentes ou cosmétiques stables et ne se séparant pas en deux phases quelle que soit la nature du tensioactif, du modificateur de rhéologie et/ou de l'agent antitartre présents.

La Demanderesse a ainsi résolu le problème du choix des agents modificateurs de rhéologie qu'ils soient dispersants ou épaississants et/ou des agents antitartres en fonction du tensioactif et permet ainsi de ne plus avoir ce problème de sélection du polymère en fonction du tensioactif.

Ainsi un des buts de l'invention est l'utilisation, pour obtenir des compositions détergentes ou cosmétiques stables quel que soit le tensioactif présent et en particulier quelle que soit la nature de l'ionicité (anionique, cationique, amphotère) ou de la non-ionicité du tensioactif présent, de copolymères répondant à la formule générale :

$$(X)a - (Y)b - (Z)c$$

dans laquelle

- X représente le motif monomérique à charge anionique constitué d'un ou plusieurs monomères éthyléniques à charge anionique
- Y représente le motif monomérique à caractère non ionique constitué d'un ou plusieurs monomères à charge non ionique
- Z représente le motif monomérique à charge cationique constitué d'un ou plusieurs monomères éthyléniques à charge cationique dont l'un au moins de ces monomères possède une structure tensioactive c'est-à-dire constituée d'une ou plusieurs chaînes latérales oxyalkylées composées de groupements alkyle, aryle, alkylaryle ou arylalkyle possédant au moins 8 atomes de carbone

et dans laquelle

a représentant le pourcentage en poids par rapport à la masse totale des monomères est compris, bornes incluses, entre 95 et 15,
b représentant le pourcentage en poids par rapport à la masse totale des monomères est compris, bornes incluses, entre 0 et 65,
c représentant le pourcentage en poids par rapport à la masse totale des monomères est compris, bornes incluses, entre 5 et 60, et plus particulièrement entre 10 et 35.

Ainsi un des buts de l'invention est la possibilité d'utiliser les dits copolymères pour obtenir des compositions détergentes ou cosmétiques stables quelle que soit la nature d'ionicité du tensioactif présent dans les formulations

contenant déjà éventuellement un modificateur de rhéologie.

Les dits copolymères utilisés selon l'invention se composent toujours d'au moins deux motifs monomériques dont le premier est constitué d'un ou plusieurs monomères éthyléniques à charge anionique, le deuxième est constitué d'un ou plusieurs monomères éthyléniques à charge cationique dont l'un au moins de ces monomères à charge cationique possède une structure tensioactive c'est-à-dire constituée d'une ou plusieurs chaînes latérales oxyalkylées composées de groupements alkyle, aryle, alkylaryle ou arylalkyle possédant au moins 8 atomes de carbone et comprenant éventuellement un troisième motif constitué d'un ou plusieurs motifs ‡ caractère non ionique, et répond à la formule générale (I) :

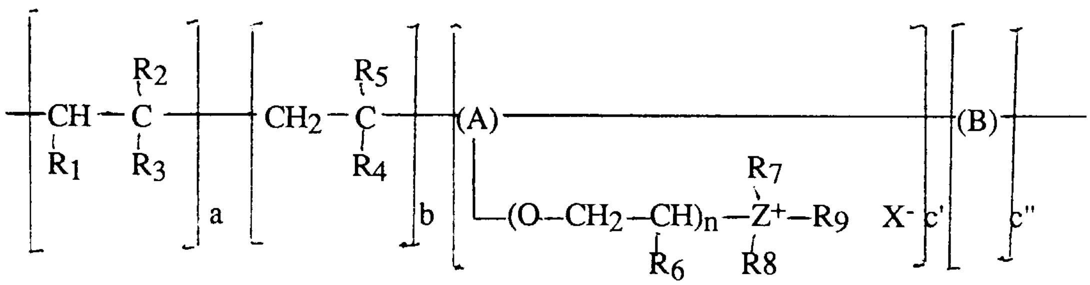

dans laquelle :

- Pour le motif de type anionique

  $R_1$ est H ou COOH éventuellement totalement ou partiellement salifié
  $R_2$ est H ou $CH_3$
  $R_3$ est un groupement comportant au moins une fonction acide éventuellement totalement ou partiellement salifiée,

  et a représentant le pourcentage en poids par rapport à la masse totale en monomère, est compris, bornes incluses, entre 95 et 15.

- Pour le motif de type non ionique

  $R_4$ est
  $-CO-NH_2$, $-CO-OR_{4'}$, $-CO-NR_{4''}R_{4'''}$,

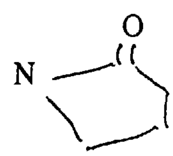

  dans lequel
  $R_4$' est un radical alkyle ou oxyalkyle ayant 1 à 4 atomes de carbone,
  $R_4$" est H ou un radical alkyle ayant 1 à 4 atomes de carbone,
  $R_4$''' est un radical alkyle ayant 1 à 4 atomes de carbone, puis
  $R_5$ est H ou $CH_3$

  et b représentant le pourcentage en poids par rapport à la masse totale en monomère, est compris, bornes incluses, entre 0 et 65.

- Pour le motif de type cationique,

  - A représente le radical insaturé polymérisable et polymérisé, appartenant au groupe des esters acrylique, méthacrylique, maléique, itaconique, crotonique, vinylphtalique ainsi que les hémiesters maléique, itaconique, vinylphtalique ou encore les insaturés uréthannes tels que par exemple les acryluréthanne, méthacryluréthanne, α-α, diméthyl-m-isopropenylbenzyluréthanne, allyluréthanne ou bien encore les éthers allyliques, les acrylamides et méthacrylamides substitués ou non, des vinyliques.

- $R_6$ est H ou $CH_3$
- $2 < n \leq 30$
- $R_7$ est un alkyle ayant 1 à 4 atomes de carbone
- Z est N ou S

X est un contre-ion sulfate ou halogénure et lorsque Z est N:

$R_8$ est une chaîne alkyle ayant 8 à 22 atomes de carbone ou un motif de formule :

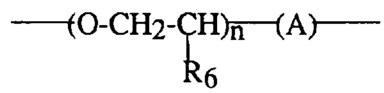

avec $2 < n \leq 30$

et

$R_9$ est une chaîne alkyle ayant 8 à 22 atomes de carbone,

et lorsque Z est S :

$R_8$ n'existe pas

$R_9$ est une chaîne alkyle ayant 8 à 22 atomes de carbone.

et c' représentant le pourcentage en poids, par rapport à la masse totale en monomère, du monomère à charge cationique possédant une structure tensioactive, est compris, bornes incluses, entre 5 et 60 et préférentiellement entre 10 et 35 avec c' + c" = c , c représentant le pourcentage en poids de tous les monomères cationiques et variant entre 5 et 60 et préférentiellement entre 10 et 35.

- B représente un motif monomérique cationique choisi parmi les monomères suivants : l'acrylate et/ou le méthacrylate de triméthyl ammonium éthyl chlorure et/ou sulfate, l'acrylamide et/ou le méthacrylamido de triméthylammonium propyl chlorure et/ou sulfate
  et c" représentant le pourcentage en poids, par rapport à la masse totale en monomère, du monomère cationique ne possédant pas la structure tensioactive, est compris, bornes incluses, entre 0 et 55 avec c'+ c" = c , c représentant le pourcentage en poids de tous les monomères à charge cationique et variant entre 5 et 60, et plus particulièrement entre 10 et 35.

Ces copolymères résultent, selon les procédés connus de la synthèse ou de la copolymérisation radicalaire en solution, en émulsion directe ou inverse, en suspension ou précipitation, en présence d'initiateurs et des régulateurs appropriés, en milieux aqueux, alcoolique, hydroalcoolique, aromatique, aliphatique ou dans un solvant halogéné d'au moins deux motifs monomériques définis ci dessus.

Ainsi, le milieu de copolymérisation peut être l'eau, le méthanol, le propanol, l'isopropanol, les butanols, ou encore le diméthylformamide, le diméthylsulfoxyde, le tétrahydrofurane, l'acétone, le méthyléthylcétone, l'acétate d'éthyle, l'acétate de butyle, l'hexane, l'heptane, le benzène, le toluène, l'éthylbenzène, le xylène, les solvants halogénés comme le tétrachlorure de carbone, le chloroforme, le dichlorométhane, les éthers de monopropylèneglycol, diéthylèneglycol.

Les copolymères destinés à être utilisés dans l'application, selon l'invention, sont généralement choisis parmi ceux ayant une viscosité spécifique comprise entre 0,3 et 10.

La viscosité spécifique des copolymères sélectionnés, qui est symbolisée par le lettre " μ " est déterminée de la manière suivante :

On prépare une solution de copolymère sous forme de sel sodique par dissolution de 50 g sec du copolymère dans un litre d'une solution d'eau distillée contenant 60 g de chlorure de sodium.

Puis, on mesure avec un viscosimètre capillaire placé dans un bain thermostaté à 25°C le temps d'écoulement d'un volume donné de la solution précitée contenant le copolymère alcalin, ainsi que le temps d'écoulement du même volume de solution aqueuse de chlorure de sodium dépourvue dudit copolymère. Il est alors possible de définir la viscosité spécifique "μ" grâce à la relation suivante :

$$\mu = \frac{(\text{Temps d'écoulement de la solution de copolymère}) - (\text{Temps d'écoulement de la solution de NaCl})}{(\text{Temps d'écoulement de la solution NaCl})}$$

Le tube capillaire est généralement choisi de telle manière que le temps d'écoulement de la solution de NaCl dépourvue de copolymère soit d'environ 90 à 100 secondes, donnant ainsi des mesures de viscosité spécifique d'une très bonne précision.

Dès la fin de la polymérisation, les copolymères acides en solution aqueuse sont recueillis et peuvent selon l'invention être mis en oeuvre sous cette forme, un agent contenu dans la formulation pouvant alors servir d'agent neutralisant.

Puis les copolymères sélectionnés en solution aqueuse sont totalement ou partiellement neutralisés par un agent de neutralisation disposant d'une fonction monovalente tel que les cations alcalins.

En pratique, la phase liquide ou hétérogène résultant de la copolymérisation et contenant le copolymère acide sélectionné peut être utilisée sous une forme salifiée comme modificateur de rhéologie et/ou agent antitartre mais elle peut également être séchée par tous les moyens connus pour en éliminer cette phase et isoler le copolymère sous la forme d'une fine poudre et être utilisée sous cette autre forme.

D'une manière générale les tensioactifs anioniques présents dans les formulations anioniques sont tels que par exemple les alkyl-benzène sulfonates parmi lesquels le tétrapropylène benzène sulfonate, les alkyl-sulfonates, les alkylsulfates, les alkyléthersulfates et autres.

De même dans les formulations cationiques les tensioactifs cationiques sont entre autres et par exemple des sels d'imidazoline, des chlorures de dialkyldiméthylammonium, des chlorures d'alkyldiméthylbenzyl ammonium.

De plus pour tous les types de formulations les tensioactifs non ioniques sont choisis par exemple parmi les éthoxylats d'alcools gras, d'alcools oxo, d'alkylphénols ainsi que les éthers d'alkyl polyglycol, d'alkylphénol polyglycol ou bien encore les alcanolamides d'acide gras ou autres.

De même les divers additifs rentrant dans la préparation des formulations sont par exemple des builders, du propylène glycol, des azurants optiques, des colorants ou autres.

La portée et l'intérêt de l'invention seront mieux perçus grâce aux exemples suivants qui ne sauraient être limitatifs.

Exemple 1 :

Cet exemple concerne la mise en évidence de la compatibilité entre divers tensioactifs et un copolymère selon l'invention de viscosité spécifique égale à 0,7.

Dans ce but pour chacun des essais on ajoute dans un bécher de 500 ml, 50 grammes d'eau, 5 grammes en poids sec du polymère à tester et 10 grammes sec du tensioactif.

Après quelques minutes d'agitation, on laisse la composition obtenue au repos pendant 24 heures.

Après ces 24 heures de repos, on observe l'aspect de la composition.

On dit que les compositions ne sont pas stables lorsqu'il y a séparation du mélange en deux phases. Au contraire on dit que les compositions obtenues sont stables lorsque que l'on observe que le mélange est toujours une solution limpide.

Dans ce cas on effectue un nouveau mélange en prenant des doses alternativement croissantes de tensioactifs et de polymère modificateur de rhéologie et/ou antitartre à tester dans les mêmes quantités d'eau jusqu'à obtenir une séparation de phases. On note alors la quantité en poids sec de tensioactif et polymère rajouté dans 50 grammes d'eau.

Les différents essais effectués sont :

Essai n° 1 :

Cet essai illustre l'art antérieur et met en oeuvre l'acide docécyl benzène sulfonique comme tensioactif et un polyacrylate de sodium de viscosité spécifique égale à 0,3 comme agent modificateur de rhéologie et plus précisément comme dispersant.

Essai n° 2 :

Cet essai illustre l'art antérieur et met en oeuvre le tensioactif de l'essai n° 1 et un polyacrylate de sodium de viscosité spécifique égale à 0,4 comme agent dispersant.

Essai n° 3 :

Cet essai illustre l'art antérieur et met en oeuvre le tensioactif de l'essai n° 1 et un polyacrylate de sodium de viscosité spécifique égale à 0,55 comme agent dispersant.

Essai n° 4 :

Cet essai illustre l'invention et met en oeuvre le tensioactif de l'essai n° 1 et un copolymère de viscosité spécifique égale à 0,7 dont la composition monomérique est

33 % en poids d'acide acrylique et 3% en poids d'acide méthacrylique pour le monomère à charge anionique

21 % en poids de monomère à charge cationique de formule générale (I) dans laquelle (A) est un radical insaturé polymérisable et polymérisé, de type méthacrylate

$R_6$ est H
n = 15-m
$R_7$ est $CH_3$
$R_8$ représente le radical -(A)- $(O\text{-}CH_2\text{-}CH_2\text{-})_m$ avec m + n = 15
$R_9$ est un radical alkyle à 12 atomes de carbone
Z = N
X = $SO_4CH_3$

43 % en poids d'acrylamide pour le monomère à caractère non ionique.

Essai n° 5 :

Cet essai illustre l'art antérieur et met en oeuvre un iso-alkylsulfate de sodium comme tensioactif et le polyacrylate de sodium de l'essai n° 1 comme agent dispersant.

Essai n° 6 :

Cet essai illustre l'art antérieur et met en oeuvre le tensioactif de l'essai n° 5 et le polyacrylate de sodium de l'essai n° 2 comme agent dispersant.

Essai n° 7 :

Cet essai illustre l'art antérieur et met en oeuvre le tensio-actif de l'essai n° 5 et le polyacrylate de sodium de l'essai n° 3 comme agent dispersant.

Essai n° 8 :

Cet essai illustre l'invention et met en oeuvre le tensioactif de l'essai n° 5 et le copolymère de l'essai n° 4.

Essai n° 9 :

Cet essai illustre l'art antérieur et met en oeuvre un alkyl éther sulfate de sodium comme tensioactif et le polyacrylate de sodium de l'essai n° 1 comme agent dispersant.

Essai n° 10 :

Cet essai illustre l'art antérieur et met en oeuvre le tensioactif de l'essai n° 9 et le polyacrylate de sodium de l'essai n° 2 comme agent dispersant.

Essai n° 11 :

Cet essai illustre l'art antérieur et met en oeuvre le tensioactif de l'essai n° 9 et le polyacrylate de sodium de l'essai n° 3 comme agent dispersant.

Essai n° 12 :

Cet essai illustre l'invention et met en oeuvre le tensioactif de l'essai n° 9 et le copolymère de l'essai n° 4.

Essai n° 13 :

Cet essai illustre l'art antérieur et met en oeuvre un alkyl phénol éthoxylé comme tensioactif et le polyacrylate de sodium de l'essai n° 1 comme agent dispersant.

Essai n° 14 :

Cet essai illustre l'art antérieur et met en oeuvre le tensioactif de l'essai n° 13 et le polyacrylate de sodium de l'essai n° 2 comme agent dispersant.

Essai n° 15 :

Cet essai illustre l'art antérieur et met en oeuvre le tensioactif de l'essai n° 13 et le polyacrylate de sodium de l'essai n° 3 comme agent dispersant.

Essai n° 16 :

Cet essai illustre l'invention et met en oeuvre le tensioactif de l'essai n° 13 et le copolymère de l'essai n° 4.

Essai n° 17 :

Cet essai illustre l'art antérieur et met en oeuvre un polyglycol éther d'alcool gras propoxylé 8 fois comme tensioactif et le polyacrylate de sodium de l'essai n° 1 comme agent dispersant.

Essai n° 18 :

Cet essai illustre l'art antérieur et met en oeuvre le tensioactif de l'essai n° 17 et le polyacrylate de sodium de l'essai n° 2 comme agent dispersant.

Essai n° 19 :

Cet essai illustre l'art antérieur et met en oeuvre le tensioactif de l'essai n° 17 et le polyacrylate de sodium de l'essai n° 3 comme agent dispersant.

Essai n° 20 :

Cet essai illustre l'invention et met en oeuvre le tensioactif de l'essai n° 17 et le copolymère de l'essai n° 4.

Essai n° 21 :

Cet essai illustre l'art antérieur et met en oeuvre un dérivé d'alkylimidazoline comme tensioactif et le polyacrylate de sodium de l'essai n° 1 comme agent dispersant.

Essai n° 22 :

Cet essai illustre l'art antérieur et met en oeuvre le tensioactif de l'essai n° 21 et le polyacrylate de sodium de l'essai n° 2 comme agent dispersant.

Essai n° 23 :

Cet essai illustre l'art antérieur et met en oeuvre le tensioactif de l'essai n° 21 et le polyacrylate de sodium de l'essai n° 3 comme agent dispersant.

Essai n° 24 :

Cet essai illustre l'invention et met en oeuvre le tensioactif de l'essai n° 21 et le copolymère de l'essai n° 4.

Le tableau n° 1 rassemble ci-dessous les quantités en grammes de tensioactif à partir des quelles il a été observé une séparation de phase dans le mélange eau, tensioactif polymère dispersant.

TABLEAU 1

| | ESSAI n° | POLYMERE | POLYMERE | TENSIOACTIF | Quantité maximale polymère + tensioactif avant séparation de phase (grammes) |
|---|---|---|---|---|---|
| | | Type | Viscosité Spécifique | Type | |
| Art antérieur | 1 | PAA Na | 0,3 | ADBS | 15 |
| | 2 | PAA Na | 0,4 | ADBS | 15 |
| | 3 | PAA Na | 0,55 | ADBS | 15 |
| Invention | 4 | Copolymère de l'essai n° 4 | 0,7 | ADBS | 20 |
| Art antérieur | 5 | PAA Na | 0,3 | Isoalkyl sulfate sodium | 40 |
| | 6 | PAA Na | 0,4 | Isoalkyl sulfate sodium | 35 |
| | 7 | PAA Na | 0,55 | Isoalkyl sulfate sodium | 35 |
| Invention | 8 | Copolymère de l'essai n° 4 | 0,7 | Isoalkyl sulfate sodium | 40 |
| Art antérieur | 9 | PAA Na | 0,3 | Alkyléthersulfate | 25 |
| | 10 | PAA Na | 0,4 | Alkyléthersulfate | 20 |
| | 11 | PAA Na | 0,55 | Alkyléthersulfate | 15 |
| Invention | 12 | Copolymère de l'essai n° 4 | 0,7 | Alkyléthersulfate | 40 |

PAA Na signifie polyacrylate de sodium

ADBS signifie acide dodécyl benzène sulfonique

TABLEAU 1 Suite

| | ESSAI n° | POLYMERE Type | POLYMERE Viscosité Spécifique | TENSIOACTIF Type | Quantité maximale polymère + tensioactif avant séparation de phase (grammes) |
|---|---|---|---|---|---|
| Art Antérieur | 13 | PAA Na | 0,3 | Alkylphénoléthoxylé | 15 |
| | 14 | PAA Na | 0,4 | Alkylphénoléthoxylé | 15 |
| | 15 | PAA Na | 0,55 | Alkylphénoléthoxylé | 15 |
| Invention | 16 | Copolymère de l'essai n° 4 | 0,7 | Alkylphénoléthoxylé | 40 |
| Art antérieur | 17 | PAA Na | 0,3 | Polyéther d'alcool gras propoxylé 8 fois | 15 |
| | 18 | PAA Na | 0,4 | Polyéther d'alcool gras propoxylé 8 fois | 15 |
| | 19 | PAA Na | 0,55 | Polyéther d'alcool gras propoxylé 8 fois | 15 |
| Invention | 20 | Copolymère de l'essai n° 4 | 0,7 | Polyéther d'alcool gras propoxylé 8 fois | 35 |
| Art antérieur | 21 | PAA Na | 0,3 | Dérivé alkylimidazoline | 25 |
| | 22 | PAA Na | 0,4 | Dérivé alkylimidazoline | 20 |
| | 23 | PAA Na | 0,55 | Dérivé alkylimidazoline | 20 |
| Invention | 24 | Copolymère de l'essai n° 4 | 0,7 | Dérivé alkylimidazoline | 40 |

PAA Na signifie polyacrylate de sodium

ADBS signifie acide dodécyl benzène sulfonique

La lecture du tableau 1 permet de constater que quel que soit le type d'ionicité du tensioactif l'usage du copolymère selon l'invention permet d'obtenir les meilleures compatibilités traduites par les plus fortes quantités de tensioactif admissibles dans le mélange sans qu'il se produise une séparation de phase.

## Revendications

**1.** Utilisation de copolymères répondant à la formule générale (I):

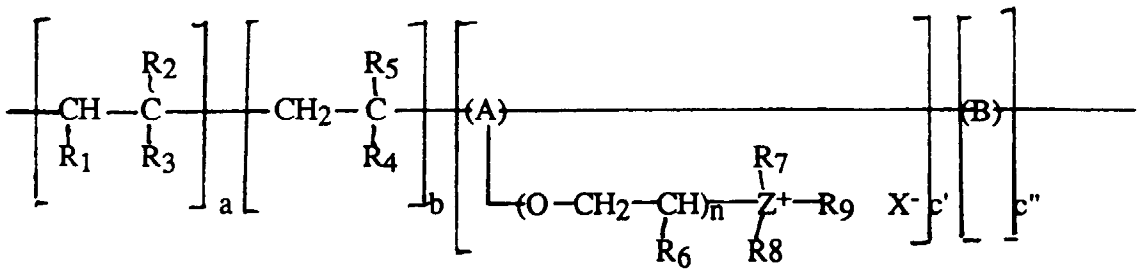

dans laquelle:

- Pour le motif de type anionique

  $R_1$ est H ou COOH éventuellement totalement ou partiellement salifié
  $R_2$ est H ou $CH_3$
  $R_3$ est un groupement comportant au moins une fonction acide éventuellement totalement ou partiellement salifiée,

  et a représentant le pourcentage en poids par rapport à la masse totale en monomère, est compris, bornes incluses, entre 95 et 15.

- Pour le motif de type non ionique

  $R_4$ est
  $-CO-NH_2$, $-CO-OR_{4'}$, $-CO-NR_{4''}R_{4'''}$,

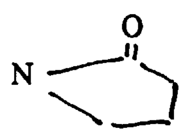

  dans lequel
  $R_{4'}$ est un radical alkyle ou oxyalkyle ayant 1 à 4 atomes de carbone,
  $R_{4''}$ est H ou un radical alkyle ayant 1 à 4 atomes de carbone,
  $R_{4'''}$ est un radical alkyle ayant 1 à 4 atomes de carbone,
  puis
  $R_5$ est H ou $CH_3$

  et b représentant le pourcentage en poids par rapport à la masse totale en monomère, est compris, bornes incluses, entre 0 et 65.

- Pour le motif de type cationique,

  - A représente le radical insaturé polymérisable et polymérisé, appartenant au groupe des esters acrylique, méthacrylique, maléique, itaconique, crotonique, vinylphtalique ainsi que les hémiesters maléique, itaconique, vinylphtalique ou encore les insaturés uréthannes tels que par exemple les acryluréthanne, méthacryluréthanne, α-α, diméthyl-m-isopropenylbenzyluréthanne, allyluréthanne ou bien encore les éthers allyliques, les acrylamides et méthacrylamides substitués ou non, des vinyliques.
  - $R_6$ est H ou $CH_3$
  - $2 < n \leq 30$
  - $R_7$ est un alkyle ayant 1 à 4 atomes de carbone
  - Z est N ou S

X est un contre-ion sulfate ou halogénure et lorsque Z est N :

$R_8$ est une chaîne alkyle ayant 8 à 22 atomes de carbone ou un motif de formule :

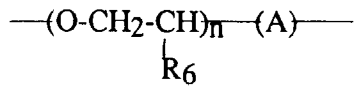

avec $2 < n \leq 30$
et
$R_9$ est une chaîne alkyle ayant 8 à 22 atomes de carbone,
et lorsque Z est S :
$R_8$ n'existe pas
$R_9$ est une chaîne alkyle ayant 8 à 22 atomes de carbone.

et c' représentant le pourcentage en poids, par rapport à la masse totale en monomère, du monomère à charge cationique possédant une structure tensioactive, est compris, bornes incluses, entre 5 et 60 avec c' + c" = c , c représentant le pourcentage en poids de tous les monomères cationiques et variant entre 5 et 60.

- B représente un motif monomérique cationique choisi parmi les monomères suivants : l'acrylate et/ou le méthacrylate de triméthyl ammonium éthyl chlorure et/ou sulfate, l'acrylamide et/ou le méthacrylamido de triméthylammonium propyl chlorure et/ou sulfate

et c" représentant le pourcentage en poids, par rapport à la masse totale en monomère, du monomère cationique ne possédant pas la structure tensioactive, est compris, bornes incluses, entre 0 et 55 avec c'+ c" = c , c représentant le pourcentage en poids de tous les monomères à charge cationique et variant entre 5 et 60.

dans le but d'obtenir des compositions détergentes ou cosmétiques stables quelle que soit la nature du tensioactif et du modificateur de rhéologie et/ou antitartre présents dans les compositions.

**2.** Utilisation du copolymère selon la revendication 1 caractérisée en ce que ledit copolymère est compatible avec les tensioactifs présents dans la formulation détergente ou cosmétique qu'ils soient anioniques, cationiques, amphotères ou non-ioniques.

**3.** Utilisation du copolymère selon l'une des revendications 1 ou 2 caractérisée en ce que ledit copolymère a une viscosité spécifique comprise entre 0,3 et 10

**4.** Utilisation du copolymère selon l'une quelconque des revendications 1 à 3 caractérisée en ce que ledit copolymère est sous forme acide.

**5.** Utilisation du copolymère selon l'une quelconque des revendications 1 à 3 caractérisée en ce que ledit copolymère est totalement ou partiellement neutralisé.

**6.** Composition détergente ou cosmétique caractérisée en ce qu'elle est stabilisée par l'utilisation du copolymère selon l'une quelconque des revendications 1 à 5.

Office européen des brevets

# RAPPORT DE RECHERCHE EUROPEENNE

Numero de la demande
EP 97 42 0144

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | GB 2 027 045 A (ROEHM GMBH) 13 février 1980<br>* page 1, ligne 27 - ligne 55; revendications 1,6,13; exemple 1 *<br>--- | 1-3,6 | C11D3/37 |
| X | GB 2 104 091 A (KAO CORP) 2 mars 1983<br>* exemples de synthèse 2,4,6,7,10 *<br>* revendications; tableau 1 *<br>--- | 6 | |
| X | JP 59 135 293 A (KAO CORP.) 3 août 1984<br>* exemple 2; tableau 1 *<br>--- | 6 | |
| A | WO 91 06623 A (UNILEVER PLC ;UNILEVER NV (NL)) 16 mai 1991<br>* page 2, ligne 27 - page 3, ligne 5 *<br>* page 10; revendications 1-6 *<br>--- | 1-6 | |
| A | EP 0 577 525 A (COATEX SA) 5 janvier 1994<br>* page 1, ligne 10 - ligne 17; revendications 1-7; exemples *<br>----- | 1 | |
| | | | DOMAINES TECHNIQUES RECHERCHES (Int.Cl.6) |
| | | | C11D |

Le présent rapport a été établi pour toutes les revendications

| Lieu de la recherche | Date d'achèvement de la recherche | Examinateur |
|---|---|---|
| LA HAYE | 16 septembre 1997 | Loiselet-Taisne, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)